# EUROPEAN PATENT APPLICATION

(11) **EP 4 616 725 A1**
(43) Date of publication of application: **17.09.2025**
(21) Application number: 23888688.1
(22) Date of filing: 07.11.2023
(51) Int. Cl.: A23L 33/135, A61K 35/745, A61P 1/00, A61P 37/02

(54) **COMPOSITION FOR NEWBORNS OR INFANTS**

(30) Priority: 07.11.2022 JP 2022178462
(71) Applicant: Morinaga Milk Industry Co., Ltd., Minato-ku Tokyo 105-7122 (JP)
(72) Inventor: XU, Chendong, Zama-shi, Kanagawa 252-8583 (JP); TANAKA, Miyuki, Zama-shi, Kanagawa 252-8583 (JP); IWABUCHI, Noriyuki, Zama-shi, Kanagawa 252-8583 (JP); MURATA, Mai, Zama-shi, Kanagawa 252-8583 (JP); HIRAKU, Akari, Zama-shi, Kanagawa 252-8583 (JP); NAKAMURA, Masahiko, Matsumoto-shi, Nagano 390-1401 (JP); TSUNO, Takahisa, Matsumoto-shi, Nagano 390-1401 (JP); NAKATA, Setsuko, Matsumoto-shi, Nagano 390-1401 (JP)
(74) Representative: Vossius & Partner Patentanwälte Rechtsanwälte mbB
(86) International application number: PCT/JP2023/040006
(87) International publication number: WO 2024/101341

(57) **Abstract**

An object is to provide a new function of *Bifidobacterium longum* subsp. *infantis* M-63 (NITE BP-02623). One kind or two or more kinds selected from bacterial cells of *Bifidobacterium longum* subsp. *infantis* M-63 (NITE BP-02623), a culture of the bacterium and a treated product of the bacterium is added to a composition taken by a healthy full-term newborn, infant or small child as an active ingredient. The composition is subjected to use for promoting colonization of a strain of *Bifidobacterium* in an intestinal microbiota, use for promoting maturation of an intestinal function, use for suppressing milk regurgitation or use for regulating immune function.

## Description

### Technical Field

The present invention relates to a composition taken by newborns, infants or small children containing a specific *Bifidobacterium* as an active ingredient.

### Background Art

Numerous bacteria inhabit in the human intestines and form a complex microbiota (flora).

*Bifidobacterium* are known to be the most dominant in the intestinal microbiota of infants and small children, and this is believed to be closely related to maintenance of the health of the infants and the small children. In general, an intestinal microbiota is a relatively stable biological system, but the intestinal microbiota is still unstable in newborns, infants and small children. In particular, the intestinal microbiota in the neonatal period is easily disturbed, and the risk of infection is high. Accordingly, establishing an intestinal microbiota in which *Bifidobacterium* are dominant in newborns, infants or small children, maintaining the intestinal microbiota and obtaining an excellent intestinal environment are important for maintaining the health of the infants or the small children.

*Bifidobacterium longum* subsp. *infantis,* which is one of the intestinal bacteria, has been attracting attention as one of probiotics, and it is reported that, when the bacterium is administered to newborns or infants, the bacterium proliferates and colonizes in the intestines, thereby improving the intestinal environment, for example by establishing an intestinal microbiota in which *Bifidobacterium* are dominant or by decreasing inflammatory cytokines in the feces (PTL 1, NPLs 1 to 7 and the like). Moreover, M-63, of *Bifidobacterium longum* subsp. *infantis,* is known to also have an action of promoting the development of the intestinal tract of an infant or a small child and an action of improving a mental health disorder of a person with the intake thereof (PTLs 2 and 3).

### Citation List

### Patent Literature

PTL 1: WO2012/092155
PTL 2: JP2021-183574A
PTL 3: WO2018/155565

### Non Patent Literature

NPL 1: Ishizeki et al., Anaerobe, 23, 38-44, (2013)
NPL 2: Roze et al., British Journal of Nutrition, 107, 1616-1622 (2012)
NPL 3: Smilowitz et al., BMC Pediatr., 17, 133, (2017)
NPL 4: Frese et al., mSphere, 2 (6), e00501-17, (2017)
NPL 5: Henrick et al., Pediatr Res., 86, 749-757, (2019)
NPL 6: J. De Andres et al., Benef Microbes, 9 (4), 573-384, (2018)
NPL 7: Escribano et al., Pediatr. Res. 83 (6), (2018)

### Summary of Invention

### Technical Problem

A problem of the invention is to provide a new function obtained when *Bifidobacterium longum* subsp. *infantis* M-63 is used as a probiotic material for newborns, infants or small children.

### Solution to Problem

As a result of extensive study to solve the problem, the present inventors have found that, in healthy full-term newborns who have taken *Bifidobacterium longum* subsp. *infantis* M-63, effects of improving the intestinal environment, such as promotion of early colonization of *Bifidobacterium* and maintenance of an intestinal microbiota in which *Bifidobacterium* are dominant in the early stage after birth, are obtained, and the invention has been thus completed.

That is, a first aspect of the invention is a composition for promoting colonization of *Bifidobacterium* in an intestinal microbiota which is a composition that contains one kind or two or more kinds selected from bacterial cells of *Bifidobacterium longum* subsp. *infantis* M-63 (NITE BP-02623), a culture of the bacterium and a treated product of the bacterium and that is taken by a healthy full-term newborn, infant or small child.

In other words, the first aspect of the invention is a composition for promoting colonization of *Bifidobacterium* in the intestinal microbiota of a healthy full-term newborn, infant or small child, containing one kind or two or more kinds selected from bacterial cells of *Bifidobacterium longum* subsp. *infantis* M-63 (NITE BP-02623), a culture of the bacterium and a treated product of the bacterium.

A second aspect of the invention is a composition for promoting maturation of an intestinal function which is a composition that contains one kind or two or more kinds selected from bacterial cells of *Bifidobacterium longum* subsp. *infantis* M-63 (NITE BP-02623), a culture of the bacterium and a treated product of the bacterium and that is taken by a healthy full-term newborn, infant or small child.

A third aspect of the invention is a composition for suppressing milk regurgitation which is a composition that contains one kind or two or more kinds selected from bacterial cells of *Bifidobacterium longum* subsp. *infantis* M-63 (NITE BP-02623), a culture of the bacterium and a treated product of the bacterium and that is taken by a healthy full-term newborn, infant or small child.

A fourth aspect of the invention is a composition for regulating immune function which is a composition that contains one kind or two or more kinds selected from bacterial cells of *Bifidobacterium longum* subsp. *infantis* M-63 (NITE BP-02623), a culture of the bacterium and a treated product of the bacterium and that is taken by a healthy full-term newborn, infant or small child.

The compositions of these aspects are preferably taken at 1.0×10⁸ to 5.0×10⁹ cfu/kg body weight/day.

The compositions of these aspects are preferably taken in combination with breast feeding.

The compositions of these aspects are preferably foods or drinks.

The compositions of these aspects are preferably pharmaceutical products.

### Advantageous Effects of Invention

According to the invention, various functions obtained when *Bifidobacterium longum* subsp. *infantis* M-63 is used as a probiotic material for a newborn, an infant or a small child are newly provided.

### Brief Description of Drawings

[FIG. 1] A figure showing the abundances of the bacteria of the groups before intake, one week after starting intake, one month of age and three months of age.
[FIG. 2] A figure showing the abundances of the genus *Bifidobacterium* bacteria of the groups before intake, one week after starting intake, one month of age and three months of age. (a) Total group, (b) vaginal delivery, and (c) cesarean section. ***:** p<0.05
[FIG. 3] A figure showing the abundances of the genus *Bifidobacterium* bacteria of the groups with and without administration of an antibiotic before intake, one week after starting intake, one month of age and three months of age. (a) With and without administration of the antibiotic, (b) without the antibiotic, and (c) with the antibiotic. *: p<0.05
[FIG. 4] A figure showing the abundances of the genus *Bifidobacterium* bacteria of the groups with a difference in taken nutrient one week after starting intake, one month of age and three months of age. (a) One week after starting intake, (b) one month of age, and (c) three months of age. *: p<0.05
[FIG. 5] A figure showing the amount of the guns *Bifidobacterium* bacteria in the feces of the groups one month of age. (a) DNA copy numbers, and (b) detection rates of the bacterial counts. *: p<0.05
[FIG. 6] A figure showing the short-chain fatty acid concentrations in the feces of the groups. (a) Before intake, and (b) one month of age. *: p<0.05
[FIG. 7] A figure showing the pH of the feces of the groups before intake and one month of age. *: p<0.05
[FIG. 8] A figure showing the IgA concentrations of the feces of the groups before intake and one month of age. *: p<0.05
[FIG. 9] A figure showing the frequencies of bowel movements and the stool consistencies of the groups one week after starting intake, one month of age and three months of age. (a) Total group, (b) breast feeding, and (c) formula and mixed feeding.
[FIG. 10] A figure showing (a) the frequencies of milk regurgitation and (b) the frequencies of vomiting of the groups one week after starting intake, one month of age and three months of age.
[FIG. 11] A figure showing (a) the periods of continuous crying for 30 minutes or longer and (b) the frequencies of continuous crying for 30 minutes or longer of the groups one week after starting intake, one month of age and three months of age.
[FIG. 12] A figure showing (a) the postpartum depression scores and (b) the changes in the postpartum depression scores of the groups before intake, one week after starting intake, one month of age and three months of age.

### Description of Embodiments

Next, the invention is explained in detail. However, the invention is not limited to the following embodiments and can be freely changed within the scope of the invention.

Here, the compositions of the first to fourth aspects of the invention are together referred to as "the composition of the invention". The composition of the invention can be in a form of a food or a drink, a pharmaceutical product or the like.

The composition of the invention contains one kind or two or more kinds selected from bacterial cells of *Bifidobacterium longum* subsp. *infantis* M-63 (NITE BP-02623), a culture of the bacterium and a treated product of the bacterium (which are sometimes together referred to as "bacterial cells and the like of *B. infantis* M-63") as an active ingredient.

*Bifidobacterium longum* subsp. *infantis* M-63 is a bacterium which was deposited for an international deposit under the Budapest Treaty on January 26, 2018 to NITE Patent Microorganisms Depositary (NPMD), National Institute of Technology and Evaluation (Room 122, 2-5-8 Kazusakamatari, Kisarazu-shi, Chiba 292-0818) and given an accession number of NITE BP-02623.

*Bifidobacterium longum* subsp. *infantis* M-63 in the present specification is not limited to the strain itself deposited or registered at the certain organization under the bacterial name (also called "deposited strain" below for the convenience of explanation) but includes substantially equivalent strains thereof (also called "derived strains" or "induced strains"). That is, the bacterium is not limited to the strain itself deposited to the depositary with the accession number but includes substantially equivalent strains thereof. The "substantially equivalent strains of the deposited strain" of the bacterium are strains which belong to the identical species as that of the deposited strain, have a genome sequence similarity (Average Nucleotide Identity value) of preferably 99.0% or more, more preferably 99.5% or more, further preferably 100% identity to the deposited strain and preferably have the identical bacteriological properties to those of the deposited strain. Examples of the substantially equivalent strains of the deposited strain of the bacterium may be strains derived from the deposited strain as the parent strain. The derived strains include a strain bred from the deposited strain and a strain naturally generated from the deposited strain. Breeding methods include modification by the genetic engineering technique and modification by mutagenesis. The mutagenesis includes X-ray irradiation, ultraviolet irradiation and treatment with a mutagen such as N-methyl-N'-nitro-N-nitrosoguanidine, ethyl methanesulfonate and methyl methanesulfonate. The strain naturally generated from the deposited strain is a strain which is naturally generated from the deposited strain during use. Such a strain is a mutant naturally generated during culture of the deposited strain (for example, passaging culture). The derived strains may be constructed with a kind of modification or constructed with two or more kinds of modification.

As the bacterium contained in the composition of the invention, a commercial product may be used. Alternatively, one appropriately produced and obtained may be used, and one obtained by culturing the bacterium described above may also be used.

The culture method is not particularly restricted as long as the bacterium can grow. As the culture method, for example, a method which is generally used for culturing lactic acid bacteria can be used directly or with appropriate modification. The culture temperature may be, for example, 25 to 50°C and is preferably 35 to 42°C. The culture can be conducted preferably under anaerobic conditions and can be conducted, for example, while flowing an anaerobic gas such as carbon dioxide. The culture can also be conducted under microaerophilic conditions such as static liquid culture. The culture can be conducted, for example, until the bacterium grows to a desired degree.

The medium used for the culture is not particularly restricted as long as the bacterium can grow. As the medium, for example, a medium which is generally used for culturing lactic acid bacteria can be used directly or with appropriate modification. That is, as a carbon source, for example, saccharides such as galactose, glucose, fructose, mannose, cellobiose, maltose, lactose, sucrose, trehalose, starch, starch hydrolysate and molasses can be used depending on the assimilation properties. As a nitrogen source, for example, ammonia and ammonium salts and nitrates such as ammonium sulfate, ammonium chloride and ammonium nitrate can be used. Moreover, as an inorganic salt, for example, sodium chloride, potassium chloride, potassium phosphate, magnesium sulfate, calcium chloride, calcium nitrate, manganese chloride, ferrous sulfate and the like can be used. Furthermore, organic components such as peptone, soybean powder, a defatted soybean cake, meat extract and yeast extract may also be used. Specific examples of the medium which is generally used for culturing *Bifidobacterium longum* subsp. *infantis* M-63 include reinforced clostridial medium, MRS medium (de Man, Rogosa, and Sharpe medium), mMRS medium (modified MRS medium), TOSP medium (TOS propionate medium), TOSP Mup medium (TOS propionate mupirocin medium), GAM (Gifu Anaerobic Medium) medium, YCFA (Yeast Extract-casein Hydrolysate Acid) medium and the like.

The form of *Bifidobacterium longum* subsp. *infantis M-*63 contained in the composition of the invention may be any of bacterial cells, a culture of the bacterium and a treated product of the bacterium.

The bacterial cells may be living bacterial cells, dead bacterial cells or a mixture of living bacterial cells and dead bacterial cells but preferably contain living bacterial cells.

The form of *Bifidobacterium longum* subsp. *infantis M-*63 contained in the composition of the invention is particularly preferably bacterial powder of living bacterial cells. The bacterial powder of living bacterial cells can be obtained by drying living bacterial cells under conditions under which the bacterium does not die.

As the culture of the bacterium, for example, the culture obtained through culture may be used directly. Alternatively, a diluted or concentrated culture may be used, or bacterial cells collected from the culture may also be used. Moreover, as the culture, a culture supernatant or a culture fraction may also be used. When a culture supernatant is used, for example, a supernatant of a culture solution obtained by culturing in GAM medium under the conditions of 37°C and 16 h can be preferably used.

As the treated product of the bacterium, crushed, heated or dried bacterial cells or culture, a diluted product thereof, a dried product thereof or a fraction thereof can be used.

A heat-treated product of bacterial cells (heat-sterilized cells) is obtained, for example, by treating the bacterium at 70 to 100°C for 10 to 40 minutes or at 90 to 150°C for five to 30 seconds. Here, pressure may be applied during the heat treatment. During the heat treatment, the temperature does not have to be constant and should be in the temperature range for a predetermined period.

As the heat-sterilized cells of the bacterium, the cells after the heat treatment may be used directly, or cells which have been subjected to treatment such as crushing, heat-drying, lyophilization and spray drying may also be used.

The amount of the bacterial cells and the like of B. *infantis* M-63 contained in the composition of the invention is not particularly limited and is appropriately determined based on the form of the composition, but for example, the bacterial cell amount of the bacterium is preferably 1×10⁴ to 1×10¹³ cfu/g or 1x10⁴ to 1×10¹³ cfu/mL, more preferably 1×10⁵ to 1×10¹² cfu/g or 1×10⁵ to 1×10¹² cfu/mL, further preferably 1×10⁶ to 1×10¹¹ cfu/g or 1×10⁶ to 1×10¹¹ cfu/mL. In the present specification, "cfu" refers to the colony forming unit. When dead bacterial cells are used, cfu/g or cfu/mL may be replaced with cells/g or cells/mL.

When a culture supernatant is used as the culture of the bacterium, the amount is preferably 0.1 to 100 mass% of the entire composition, more preferably 1 to 90 mass%, further preferably 10 to 80 mass%.

The amount of the bacterial cells and the like of B. *infantis* M-63 contained in the entire composition of the aspect is preferably 0.001 mass% or more and less than 100 mass%, more preferably 0.005 to 95 mass%, further preferably 0.01 to 85 mass%.

The ranges may be generally ranges of amount for distribution as an oral composition.

The composition of the first aspect of the invention has an action of promoting colonization of a strain of *Bifidobacterium* in an intestinal microbiota and is thus used for obtaining the effect. The Bifidobacterium which colonize in the intestinal microbiota are not limited to *Bifidobacterium longum* subsp. *infantis* M-63 and may include other *Bifidobacterium longum* subsp. *infantis* strains, *Bifidobacterium longum* subsp. *longum, Bifidobacterium breve, Bifidobacterium bifidum, Bifidobacterium adolescentis, Bifidobacterium catenulatum, Bifidobacterium pseudocatenulatum, Bifidobacterium animalis, Bifidobacterium lactis, Bifidobacterium pseudolongum* and the like, and some of the species or the strains need only to be colonized. Here, the "colonization" means that the bacterial species exist in the intestinal microbiota for a long time, preferably for one month or longer, more preferably three months or longer. The colonization further preferably means that Bifidobacterium are detected at 1.0×10⁶ cells/g feces or more from the intestinal microbiota even one month or longer after the intake of the composition of the invention.

It can be determined that colonization of the Bifidobacterium in an intestinal microbiota is promoted when the existence proportion of the Bifidobacterium in the intestinal microbiota increases compared to that without the intake of the composition of the aspect or that before the intake of the composition of the aspect. The "existence proportion" can be replaced with the "abundance" in the entire bacterial group detected in the intestinal microbiota. Here, as long as the existence proportion of the Bifidobacterium in the intestinal microbiota increases, a case in which the existence proportion of other bacteria in the intestinal microbiota decreases may be included.

It can be determined that colonization of the Bifidobacterium in an intestinal microbiota is promoted also when the existence amount of the Bifidobacterium in the intestinal microbiota increases compared to that without the intake of the composition of the aspect or that before the intake of the composition of the aspect. The "existence amount" can be determined by measuring the bacterial count of the Bifidobacterium contained in feces by a general method.

It can be determined that colonization of the Bifidobacterium in an intestinal microbiota is promoted also when the intestinal acetic acid concentration increases and/or when the intestinal pH decreases compared to that without the intake of the composition of the aspect or that before the intake of the composition of the aspect. In general, when Bifidobacterium increase, the amount of acetic acid that the strains produce increases, and as a result, the intestinal pH decreases. Here, the acetic acid concentration and/or the pH can be measured by analyzing feces by a general method.

When the colonization of the Bifidobacterium in an intestinal microbiota is promoted by the composition of the aspect, the intestinal microbiota is adjusted to an excellent environment at an early stage.

When the intestinal microbiota becomes an excellent environment due to the composition of the aspect, in the subject of intake (administration), for example, effects such as suppression of increase in bacteria such as *Escherichia coli* in the intestines, intestinal regulation, relief from constipation, improvement of intestinal tract barrier function, suppression of inflammation of the intestinal tract, relief, alleviation or improvement of a symptom of irritable bowel syndrome (IBS) or ulcerative colitis (UC), improvement of metabolism of dietary fiber, normal development of immune function and improvement of a symptom of atopic eczema in infancy can also be obtained.

The effect of promoting colonization of the Bifidobacterium in an intestinal microbiota of the aspect can be exhibited, regardless of whether or not an antibiotic has been administered to the mother of the subject of intake during delivery.

The composition of the second aspect of the invention has an action of promoting maturation of an intestinal function and is thus used for obtaining the effect. The intestinal function here includes water-absorbing function and nutrient metabolism function.

It can be determined that maturation of an intestinal function is promoted when the stool consistency or the frequency of bowel movements improves compared to that without the intake of the composition of the aspect or that before the intake of the composition of the aspect. Specifically, it can be determined when watery stools become less or when the frequency of bowel movements decreases in the normal range.

The composition of the third aspect of the invention has an action of suppressing milk regurgitation and is thus used for obtaining the effect. Milk regurgitation is spitting up of a small amount of milk from the mouth of an infant after feeding.

It can be determined that milk regurgitation is suppressed when the number or the frequency of milk regurgitation decreases compared to that without the intake of the composition of the aspect or that before the intake of the composition of the aspect.

The composition of the fourth aspect of the invention has an action of regulating immune function and is thus used for obtaining the effect. Here, the "immune regulation" refers to acquiring balance so that immunity functions appropriately and includes "immunostimulation" and "anti-inflammation". The "immunostimulation" refers to the action towards facilitation of immunoreaction and specifically includes an action of improving decreased immunoreaction and an action of further enhancing normal or satisfactory immunoreaction. Moreover, the action towards facilitation of immunoreaction may include actions of protecting against a viral infection and suppressing viral proliferation (together also referred to as an antiviral action) which are caused by immunostimulation, an action of treating or preventing an infectious disease and an action of improving/alleviating a symptom of the disease. Here, "prevention" refers to prevention or delay of the onset of a disease or a symptom in the subject of application or a decrease in the risk of a disease or a symptom of the subject of application. Moreover, the "anti-inflammation" refers to an action towards suppression of inflammation (anti-inflammatory action) to prevent immunoreaction from being excessive.

It can be determined that immune function is regulated when the amount of an immunoglobulin such as IgA in the intestines increases and/or when the amount of an inflammatory cytokine such as IL-1β, IL-2, IL-5, IL-6, IL-8 and IL-22 decreases compared to that without the intake of the composition of the aspect or that before the intake of the composition of the aspect.

Further, in a fifth aspect of the invention, a composition that contains one kind or two or more kinds selected from bacterial cells of *Bifidobacterium longum* subsp. *infantis* M-63 (NITE BP-02623), a culture of the bacterium and a treated product of the bacterium and that is taken by a healthy full-term newborn, infant or small child is also provided, and the composition also exhibits an effect of suppressing postpartum depression of a woman after delivery. Thus, the composition can be used for suppressing postpartum depression of a woman after delivery (a mother after delivery).

The subject who takes the composition of the aspect is a newborn, an infant or a small child, and as in the Examples shown below, it has been found that milk regurgitation of the subject of intake is suppressed and that the period and the frequency of continuous crying decrease. Thus, it is speculated that postpartum depression is suppressed because postpartum stress on the woman after delivery who nurses the subject of intake is relieved and because the woman feels better.

It can be determined that postpartum depression is suppressed when the index for evaluating postpartum depression, such as the scores of Edinburgh Postnatal Depression Scale (EPDS), decreases or when the increase thereof is suppressed compared to that without the intake of the composition of the aspect or that before the intake of the composition of the aspect.

The subject to which the composition of the invention is administered (or which takes the composition) is not particularly limited as long as the subject is an animal but is generally a mammal, preferably a human. The subject to which the composition of the invention is administered (or which takes the composition) is a healthy full-term newborn, infant or small child, preferably a newborn or an infant, more preferably a newborn. When a nonhuman animal is the subject, the child may be synonymous with a young animal.

Here, a healthy full-term child refers to a child born at 37 weeks or later having a birth weight of 2500 g or more. That is, the child refers to a child excluding low birth weight infants and pre-term infants in which the degree of development of the intestines is different.

Moreover, a newborn refers to a child which is younger than four weeks old, and an infant refers to a child who is 28 days old or older and younger than one year old. A small child refers to a child between one year old and before entering school or before seven years old.

The composition of the invention may be used for therapeutic purpose or may be used for non-therapeutic purpose.

The "non-therapeutic purpose" is a concept which does not include medical practice, namely treatment of a human body by therapy. Examples thereof include health promotion, beauty treatment and the like.

The "therapeutic purpose" means for treatment of a disease, improvement of a symptom or prevention of a disease or a symptom. The "improvement" means: improvement of a disease, a symptom or the condition; prevention or delay of deterioration of a disease, a symptom or the condition; or reversal, prevention or delay of progress of a disease or a symptom. The "prevention" means prevention or delay of the onset of a disease or a symptom in the subject of the application or reduction of the risk of a disease or a symptom of the subject of the application.

When the composition of the invention is used for therapeutic purpose, the composition can be administered to a non-healthy individual as the subject.

The non-healthy individual is a human without having an excellent intestinal microbiota, a human having a disease or a symptom caused by abnormality of the intestinal microbiota, a human having immature intestinal functions, a human (especially an infant) who easily causes milk regurgitation, a human having abnormality of immune function, a human having a disease or a symptom caused by abnormality of immune function, a human having an infectious disease or the like. The disease or the symptom caused by abnormality of the intestinal microbiota may be constipation, inflammation of the intestinal tract, irritable bowel syndrome (IBS), ulcerative colitis (UC), atopic eczema in infancy or the like.

When the composition of the invention is used for therapeutic purpose, the colonization of the Bifidobacterium is promoted, and the intestinal microbiota can be adjusted to an excellent environment at an early stage. Maturation of an intestinal function is promoted, or milk regurgitation is suppressed. Alternatively, immune function is regulated. Moreover, a disease or a symptom caused by abnormality of the intestinal microbiota or abnormality of immune function or an infectious disease can be improved, prevented or treated.

When the composition of the invention is used for non-therapeutic purpose, the composition can be administered to a healthy individual as the subject.

When the composition of the invention is used for non-therapeutic purpose, the colonization of the Bifidobacterium is promoted, and the intestinal microbiota can be adjusted to an excellent environment at an early stage. When the intestinal microbiota becomes an excellent environment, a health-promoting effect such as suppression of increase in bacteria such as *Escherichia coli* in the intestines, intestinal regulation, relief from constipation, improvement of intestinal tract barrier function, suppression of inflammation of the intestinal tract, improvement of metabolism of dietary fiber and normal development of immune function can also be obtained. Moreover, a health-promoting effect of regulating immune function or improving resistance to a virus can also be obtained.

Moreover, postpartum depression can be suppressed because postpartum stress on the woman after delivery who nurses the subject (mainly an infant or a small child) of intake of the composition of the invention is relieved and because the woman feels better.

Another aspect of the first aspect of the invention is a composition for promoting colonization of a strain of *Bifidobacterium* in the intestinal microbiota of a healthy full-term newborn, infant or small child containing the bacterial cells and the like of *B. infantis* M-63.

Another aspect of the first aspect of the invention is use of the bacterial cells and the like of *B. infantis* M-63 for the manufacture of a composition for promoting colonization of a strain of *Bifidobacterium* in the intestinal microbiota of a healthy full-term newborn, infant or small child.

Another aspect of the first aspect of the invention is use of the bacterial cells and the like of *B. infantis* M-63 in promoting colonization of a strain of *Bifidobacterium* in the intestinal microbiota of a healthy full-term newborn, infant or small child.

Another aspect of the first aspect of the invention is the bacterial cells and the like of *B. infantis* M-63 for use in the promotion of colonization of a strain of *Bifidobacterium* in the intestinal microbiota of a healthy full-term newborn, infant or small child.

Another aspect of the first aspect of the invention is a method for promoting colonization of a strain of *Bifidobacterium* in an intestinal microbiota including administering the bacterial cells and the like of *B*. *infantis* M-63 to a healthy full-term newborn, infant or small child.

Another aspect of the second aspect of the invention is a composition for promoting maturation of an intestinal function of a healthy full-term newborn, infant or small child containing the bacterial cells and the like of *B*. *infantis* M-63.

Another aspect of the second aspect of the invention is use of the bacterial cells and the like of *B. infantis* M-63 for the manufacture of a composition for promoting maturation of an intestinal function of a healthy full-term newborn, infant or small child.

Another aspect of the second aspect of the invention is use of the bacterial cells and the like of *B. infantis* M-63 in promoting maturation of an intestinal function of a healthy full-term newborn, infant or small child.

Another aspect of the second aspect of the invention is the bacterial cells and the like of *B. infantis* M-63 for use in the promotion of maturation of an intestinal function of a healthy full-term newborn, infant or small child.

Another aspect of the second aspect of the invention is a method for promoting maturation of an intestinal function including administering the bacterial cells and the like of *B. infantis* M-63 to a healthy full-term newborn, infant or small child.

Another aspect of the third aspect of the invention is a composition for suppressing milk regurgitation of a healthy full-term newborn, infant or small child containing the bacterial cells and the like of *B. infantis* M-63.

Another aspect of the third aspect of the invention is use of the bacterial cells and the like of *B. infantis* M-63 for the manufacture of a composition for suppressing milk regurgitation of a healthy full-term newborn, infant or small child.

Another aspect of the third aspect of the invention is use of the bacterial cells and the like of *B. infantis* M-63 in suppressing milk regurgitation of a healthy full-term newborn, infant or small child.

Another aspect of the third aspect of the invention is the bacterial cells and the like of *B. infantis* M-63 for use in the suppression of milk regurgitation of a healthy full-term newborn, infant or small child.

Another aspect of the third aspect of the invention is a method for suppressing milk regurgitation including administering the bacterial cells and the like of *B*. *infantis* M-63 to a healthy full-term newborn, infant or small child.

Another aspect of the fourth aspect of the invention is a composition for regulating the immune function of a healthy full-term newborn, infant or small child containing the bacterial cells and the like of *B. infantis* M-63.

Another aspect of the fourth aspect of the invention is use of the bacterial cells and the like of *B. infantis* M-63 for the manufacture of a composition for regulating the immune function of a healthy full-term newborn, infant or small child.

Another aspect of the fourth aspect of the invention is use of the bacterial cells and the like of *B. infantis* M-63 in regulating the immune function of a healthy full-term newborn, infant or small child.

Another aspect of the fourth aspect of the invention is the bacterial cells and the like of *B. infantis* M-63 for use in the regulation of the immune function of a healthy full-term newborn, infant or small child.

Another aspect of the fourth aspect of the invention is a method for regulating immune function including administering the bacterial cells and the like of *B*. *infantis* M-63 to a healthy full-term newborn, infant or small child.

Here, "administering the bacterial cells and the like of *B. infantis* M-63 to a subject child" may be synonymous with "causing a subject child to take the bacterial cells and the like of *B. infantis* M-63". The intake may be voluntary (free intake) or may be forced (forced intake). That is, the administration step may be specifically, for example, a step of blending the bacterial cells and the like of *B. infantis* M-63 in a food, a drink or feed for supplying to a subject child and thus causing the subject child to freely take the bacterial cells and the like of ***B.** infantis* M-63.

The timing of intake (administration) of the composition of the invention and the intake (administration) period are not particularly limited, but the intake (administration) is preferably started on the seventh day after birth or later. Moreover, the composition is taken (administered) continuously preferably for one week or longer, more preferably one month or longer, further preferably three months or longer.

The composition of the invention itself may be in the form of a food, a drink, a pharmaceutical product or the like or may be in a form which is contained in a food, a drink, a pharmaceutical product or the like as an additive.

The route of intake (administration) of the composition of the invention may be an oral or parenteral route but is generally an oral route. The parenteral intake (administration) is transdermal, intravenous or rectal administration, inhalation or the like.

The intake (dosage) of the composition of the invention is appropriately selected based on the age of the subject of intake (administration), the gender, the condition, other conditions and the like.

The intake (dosage) of the composition of the invention is, as the intake (dosage) of *Bifidobacterium longum* subsp. *infantis* M-63 according to the invention, for example, preferably 1.0×10⁸ to 5.0×10⁹ cfu/kg body weight/day, more preferably 5.0×10⁸ to 5.0×10⁹ cfu/kg body weight/day, further preferably 1.7×10⁹ to 3.3×10⁹ cfu/kg body weight/day. The effects described above of the composition of the invention are obtained even at such a low intake (dosage), compared to those of conventional probiotics using Bifidobacterium, and the composition is useful because the burden to the subject child is thus small.

Regardless of the amount or the period of intake (administration), the composition of the invention can be taken (administered) once a day or in multiple divided portions.

The composition of the invention, especially the composition of the first or second aspect, is preferably taken in combination with breast feeding when the subject child is in the lactation period.

By receiving breast feeding, breast milk components such as human milk oligosaccharides are taken, and it is believed that the actions of *B. infantis* M-63 are further enhanced and that colonization of the Bifidobacterium in an intestinal microbiota is further improved.

As long as breast feeding is combined, mixed feeding with a formula is acceptable, but the colonization effect is obtained more easily when the proportion of breast feeding is higher.

Here, "breast feeding" refers to feeding breast milk to a newborn, an infant or a small child for nutrition.

When the composition of the invention is an orally taken composition, the composition is preferably a food or a drink in an embodiment.

The form and the property of the food or the drink are not particularly restricted as long as the food or the drink does not impair the effects of the invention and can be orally taken, and the food or the drink can be produced by a general method using a material which is generally used for a food or a drink except that the bacterial cells and the like of *B. infantis* M-63 are contained.

Although appropriate selection is necessary according to the age of the subject child, the food or the drink is not limited regarding the form such as liquid, paste, gel solid or powder. Examples thereof include the following examples: tablet candies; liquid foods (nutrition products for tube feeding); wheat products such as breads, macaroni, spaghetti, noodles, cake mixes, frying flours and bread crumbs; instant foods such as instant noodles, cup noodles, retort-pouched/prepared foods, prepared canned foods, microwave foods, instant soups/stews, instant miso soups/clear Japanese soups, canned soups, freeze-dried foods and other instant foods; processed agricultural products such as canned agricultural products, canned fruits, jams/marmalades, pickles, cooked beans, dried agricultural products and cereals (processed grains); processed fishery products such as canned fishery products, fish hams/sausages, fishery paste products, fishery delicacies and *Tsukudani* (foods boiled down in sweetened soy sauce); processed livestock products such as canned livestock products/pastes and livestock hams/sausages; milk/dairy products such as processed milk, milk beverages, yogurts (fermented milk), lactic acid bacteria beverages, cheeses, ice creams, creams and other dairy products; oils and fats such as butter, margarine and vegetable oils; basic condiments such as soy sauce, soybean paste, sauces, processed tomato condiments, *Mirin* (sweet sake for seasoning) and vinegars; compound flavor enhancers/foods such as cooking mixes, curry roux, sauces, dressings, noodle broths, spices and other compound flavor enhancers; frozen foods such as frozen food materials, semi-cooked frozen foods and cooked frozen foods; confectioneries such as caramels, candies, chewing gums, chocolates, cookies, biscuits, cakes, pies, snacks, crackers, Japanese-style confectioneries, rice confectioneries, bean confectioneries, desserts, jellies and other confectioneries; luxury beverages such as carbonated drinks, natural juices, fruit juices, fruit juice-containing soft drinks, fruit flesh drinks, fruit granule-containing fruit juices, vegetable drinks, soy milk, soy milk drinks, coffee drinks, tea drinks, drink powders, concentrated drinks, sport drinks, nutritional drinks, alcohols and other luxury beverages, other commercial foods such as baby foods, *Furikake* (dry Japanese seasonings) and seasonings for *Chazuke* (boiled rice with hot tea) and the like; nutritional compositions such as supplements and formulas (including powdered milk, liquid milk and the like); enteral nutrition products; functional foods (foods for specified health uses and foods with nutrient function claims); and the like.

As described above, the food composition of the invention can be administered to any subject including heathy individuals and non-healthy individuals but is used for the non-therapeutic purpose when the composition is a food or a drink labeled with a specific use (especially a health use) or a function.

Of these, a nutritional composition is preferable.

In the invention, the "nutritional composition" is not particularly limited as an aspect of the food or the drink but is preferably a formula, a liquid food or the like, more preferably a formula. The subject of intake may be an infant, a small child, a child or an adult but is preferably an infant or a small child.

The formulas include a powdered formula and a liquid formula.

The powdered formula is defined by the Ministerial Ordinance Concerning Compositional Standards, etc. for Milk and Milk Products as "those obtained by processing raw milk, cow's milk, certified milk or a food produced therefrom as a raw material or using such a material as a major raw material, adding nutrients necessary for infants and small children and processing into powder".

The liquid formula is defined by the Ministerial Ordinance as "those obtained by processing raw milk, cow's milk, certified milk or a food produced therefrom as a raw material or using such a material as a major raw material, adding nutrients necessary for infants and small children and processing into liquid".

The formulas also include those which contain a nutrient component such as proteins, oils and fats, carbohydrates, minerals and vitamins and which are processed into powder or liquid.

The formulas further include "a powdered infant formula", "a liquid infant formula" and "a powdered formula for pregnant and parturient women and nursing mothers" of the food for special dietary uses provided by the Health Promotion Act and also include aspects as a powdered infant formula for small children, nutritional powder for adults, nutritional powder for the aged and the like.

When the composition of the invention is in a supplement form, the supplement can be formulated into a solid preparation such as powder, granules, tablets and capsules, a liquid preparation such as a solution, a syrup, a suspension and an emulsion or the like. The formulation can be in accordance with the explanations for the components, the carrier and the method for formulation of the pharmaceutical product described below.

An embodiment of the food or the drink can be feed. The feed is pet food, livestock feed, fish farming feed or the like.

The form of the feed is not particularly restricted, and the feed may contain, in addition to the bacterial cells and the like of *B. infantis* M-63, for example the following materials: grain such as corn, wheat, barley, rye and milo; vegetable oil cake such as soybean oil cake, rapeseed oil cake, coconut oil cake and linseed oil cake; bran such as wheat bran, barley bran, rice bran and defatted rice bran; a food manufacturer's by-product such as corn gluten meal and corn jam meal; animal feed such as fish powder, skim milk powder, whey, yellow grease and tallow; yeast such as torula yeast and brewer's yeast; mineral feed such as tertiary calcium phosphate and calcium carbonate; an oil or a fat; a single amino acid; a saccharide; or the like.

When the composition of the invention is an embodiment of a food or a drink (including feed), the composition can be provided/sold as a food or a drink labeled with use related to improvement of the intestinal microbiota or the like. Moreover, the bacterial cells and the like of B. *infantis* M-63 according to the present specification can be used for manufacturing the foods, the drinks and the like.

The "labeling" act includes all the acts for informing a consumer of the use, and all the expressions which can remind of/cause to guess the use are the "labeling" acts of the invention, regardless of the purposes of labeling, the contents of labeling, the objects to be labeled, the media and the like.

The "label" is preferably with an expression which allows a consumer to directly recognize the use. Specific examples include an act of transferring an article in which the use is described on a product regarding the food or the drink or packaging of a product, delivering such an article, displaying such an article for transfer or delivery or importing such an article, an act of displaying or distributing an advertisement of a product, a price list or a business document with a description of the use thereon or providing information with such contents with a description of the use by an electromagnetic method (the Internet or the like) and another act.

The content of the label is preferably a label approved by the administration or the like (for example, a label approved based on a system provided by the administration and provided in the form based on the approval or the like). It is preferable to label with such a content on packaging, a container, a catalogue, a brochure, an advertisement material in a sales site such as POP, other documents or the like.

The "labels" also include labels with health foods, functional foods, enteral nutrition products, food for special dietary uses, food with health claims, foods for specified health uses, foods with nutrient function claims, foods with function claims, quasi-drugs and the like. In particular, the labels are labels approved by the Consumer Affairs Agency, such as labels approved by the systems for foods for specified health uses, foods with nutrient function claims or foods with function claims and labels approved by a similar system. Specific examples include a label with foods for specified health uses, a label with qualified foods for specified health uses, a label indicating influence on the structure or the function of a body, a label with reduction of disease risk, a label with a scientifically grounded function and the like. More specifically, typical examples include labels with food for specified health uses (especially labels with health uses) provided by the Cabinet Office Ordinance on Labeling Permission for Special Dietary Uses under the Health Promotion Act (Cabinet Office Ordinance No. 57 on August 31, 2009) and similar labels.

The labels are, for example, labels with "increasing good bacteria in the stomach of infants and small children", "contributing to health of the stomach of infants and small children", "supporting immune function of infants and small children" and the like.

In an embodiment, the composition of the invention can be a pharmaceutical product.

The administration route of the pharmaceutical product may be an oral or parenteral route but is preferably an oral route. The parenteral intake (administration) is transdermal, intravenous or rectal administration, inhalation or the like.

Regarding the form of the pharmaceutical product, the composition can be appropriately formulated into a desired dosage form depending on the administration method. For example, in the case of oral administration, the composition can be formulated into a solid preparation such as powder, granules, tablets and capsules, a liquid preparation such as a solution, a syrup, a suspension and an emulsion or the like. The composition can also be formed into an enteric-coated agent by enteric coating or the like. In the case of parenteral administration, the composition can be formulated into a suppository, ointment, an injection or the like.

For the formulation, a component which is generally used for formulation such as excipients, pH-adjusting agents, colorants and corrigents can be used in addition to the bacterial cells and the like of *B. infantis* M-63. Another medicinal component, a component having an action of improving an intestinal microbiota which is known or will be found in the future or the like can also be used in combination.

In addition, the formulation can be appropriately conducted by a known method depending on the dosage form. For the formulation, a carrier which is generally used for formulation may be appropriately blended and formulated. Such carriers are excipients, binders, disintegrating agents, lubricants, stabilizers, flavoring agents and the like.

As described above, the pharmaceutical product of the invention can be administered to any subject including healthy individuals and non-healthy individuals, but in the case of a pharmaceutical product for treating a disease or relieving a symptom, the pharmaceutical product is used for the therapeutic purpose for a non-healthy individual while, in the case of a pharmaceutical product for preventing a disease or a symptom, the pharmaceutical product is used for the therapeutic purpose for a healthy individual.

Examples of the excipients include: saccharide derivatives such as lactose, sucrose, glucose, mannitol and sorbitol; starch derivatives such as cornstarch, potato starch, α-starch, dextrin and carboxymethyl starch; cellulose derivatives such as crystalline cellulose, hydroxypropyl cellulose, hydroxypropyl methylcellulose, carboxymethylcellulose and carboxymethyl cellulose calcium; gum arabic; dextran; pullulan; silicate derivatives such as light silicic anhydride, synthetic aluminum silicate and magnesium aluminometasilicate; phosphate derivatives such as calcium phosphate; carbonate derivatives such as calcium carbonate; sulfate derivatives such as calcium sulfate; and the like.

Examples of the binders include, in addition to the excipients, gelatin, polyvinyl pyrrolidone, macrogol and the like.

Examples of the disintegrating agents include, in addition to the excipients, chemically modified starch or cellulose derivatives such as croscarmellose sodium, sodium carboxymethyl starch and cross-linked polyvinyl pyrrolidone and the like.

Examples of the lubricants include: talc; stearic acid; metal stearates such as calcium stearate and magnesium stearate; colloidal silica; waxes such as Veegum and spermaceti wax; boric acid; glycols; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acid such as silicic anhydride and silicic acid hydrate; starch derivatives; and the like.

Examples of the stabilizers include: paraoxybenzoate esters such as methylparaben and propylparaben; alcohols such as chlorobutanol, benzyl alcohol and phenylethyl alcohol; benzalkonium chloride; acetic anhydride; sorbic acid; and the like.

Examples of the flavoring agents include sweeteners, acidulants, aromas and the like.

In this regard, the carrier used in the case of a liquid preparation for oral administration is a solvent such as water or the like.

The timing for taking the pharmaceutical product of the invention is not particularly limited, and examples include before a meal, after a meal, between meals, before bedtime and the like.

### Examples

The invention is explained further specifically below using Examples, but the invention is not limited to the Examples.

### <Test Example> Clinical Trial of Administration of B. infantis M-63 to Newborns

Healthy full-term newborns were divided into two groups: one thereof was named an M-63 group (analysis subjects n=56); and the other was named a placebo group (analysis subjects n=53). The M-63 group continuously took bacterial powder of living bacterial cells of *Bifidobacterium longum* subsp. *infantis* M-63 (manufactured by Morinaga Milk Industry Co., Ltd.) at one billion cells/day, and the placebo group continuously took 1 g/day of sterilized dextrin, each until the age of three months. Examination was conducted before the intake (until the seventh day after birth), one week after starting the intake, one month of age and three months of age. The contents of the examination were collection of fecal samples, questionnaires on the states of the subject children (including frequency of bowel movements, frequency of milk regurgitation/vomiting and stopping of crying) and Edinburgh Postnatal Depression Scale (EPDS) of the mothers. The collected fecal samples were stored at -80°C until the analysis.

### (1) Measurement of Abundances and Amounts of Bifidobacterium in Intestinal Microbiota

The abundances of bacterial phyla in the intestinal microbiota were measured by the following procedures. DNA was extracted from the fecal samples according to the method of Sugahara *et al.* (Sugahara , H., et al., Biosci. Microbiota. Food. Health. 2015,34:87-93). Amplification of the extracted DNA and analysis of the sequences by Illumina Miseq (Illumina, Inc., San Diego, CA, USA) were conducted according to the method of Odamaki *et al.* (Odamaki, T., et al., Sci. Rep. 2018, 8:85). From the obtained paired-end sequences, the sequences matching Genome Reference Consortium human build 38 (GRCh38) and phiX were removed, and then the microbiotas were analyzed with QIIME2 software (version 2017.10 (https://qiime2.org/)) as shown below. Through the DADA2 pipeline, the sequences of the 30 bases at the 3' side of the forward sequence and the 90 bases at the 3' end of the reverse sequence were trimmed, and the error sequences were removed. Using Naive Bayes classifier algorism using Greengenes13.8 as the training data, the sequences having a homology of 99% were considered as OTUs (Operational Taxonomy Units), and the phylogenetic classification was estimated.

The bacterial counts of Bifidobacterium in the fecal samples were quantified by the following procedures. Quantitative PCR reaction solutions containing a primer set for detecting a sequence specific to Bifidobacterium were prepared. Using Applied Biosystems 7500 real-time PCR system (Thermo Fisher SCIENTIFIC), real-time PCR was conducted under the conditions of heating at 95°C for 20 seconds, then denaturation at 95°C for three seconds, annealing/elongation at 60°C for 30 seconds and 40 cycles. Here, the detection limit is 10⁶ copies/g feces.

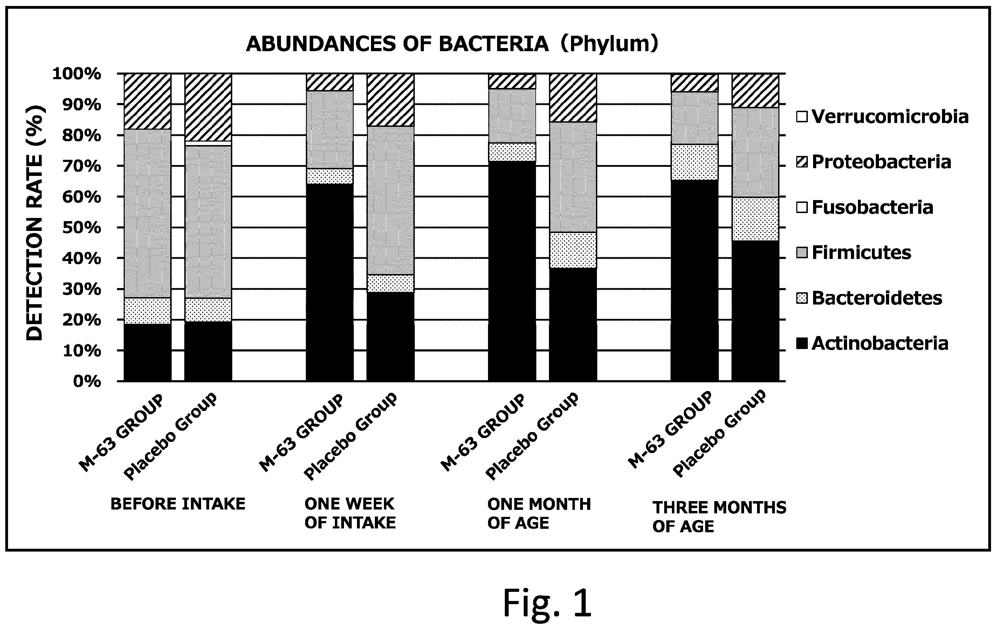

The results are shown in Figs. 1 to 5.

As shown in Fig. 1, the abundance of Actinobacteria (including Bifidobacterium) was higher, and the abundances of Firmicutes and Proteobacteria were significantly lower (p<0.05) in the M-63 group than those of the placebo group one week after starting the intake, one month of age and three months of age.

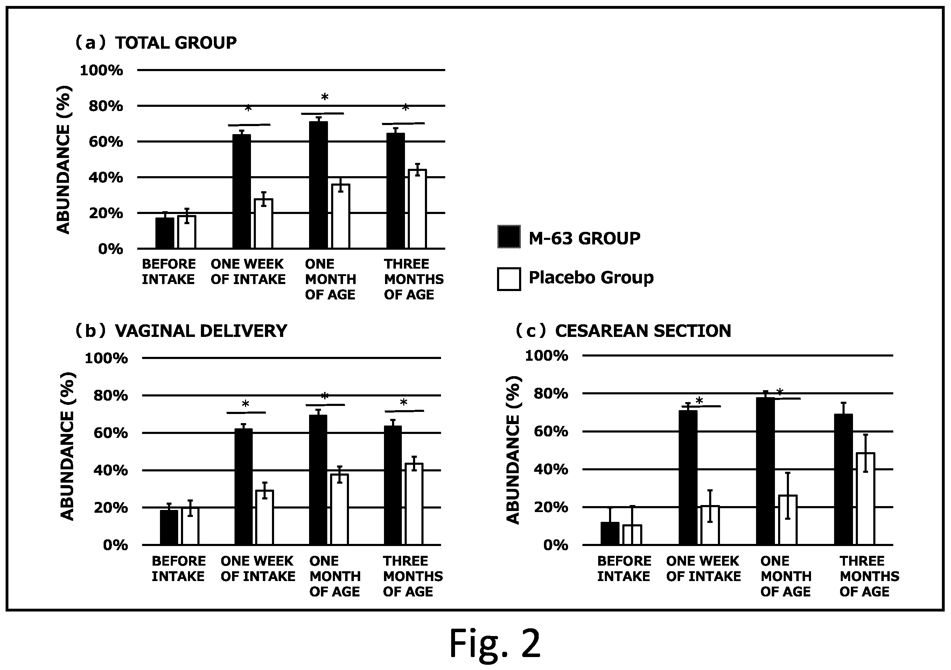

As shown in Fig. 2, the abundance of the Bifidobacterium was higher in the M-63 group than that of the placebo group one week after starting the intake, one month of age and three months of age. Among the newborns born with cesarean section, the abundance of the Bifidobacterium before the intake was low, and the difference between the M-63 group and the placebo group was large one week after starting the intake and one month of age, compared to that in the newborns born by vaginal delivery.

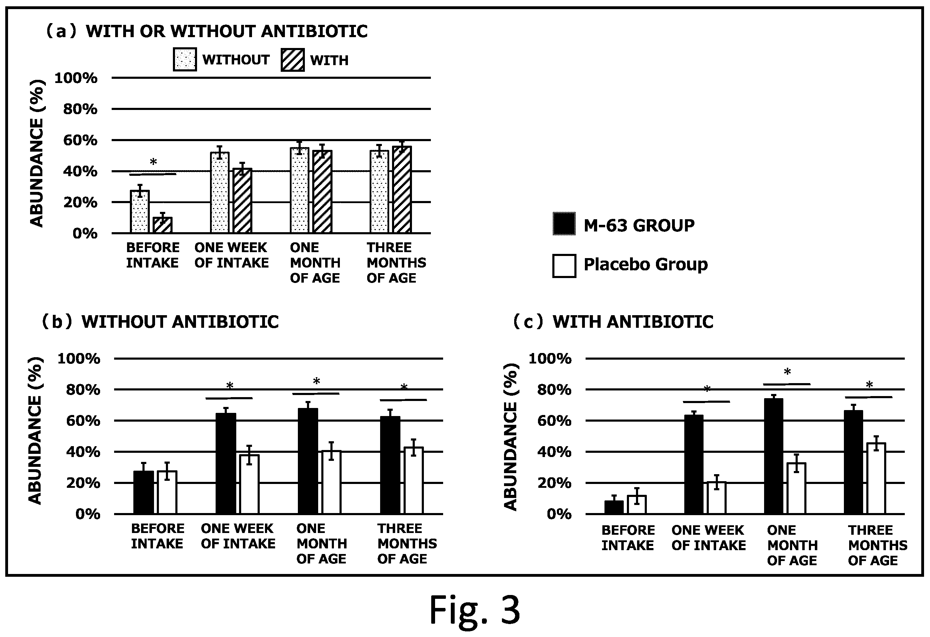

As shown in Fig. 3, the abundance of the Bifidobacterium before the intake was low when the mothers used an antibiotic during the delivery. Irrelevant to the use of an antibiotic by the mothers, the abundance of the Bifidobacterium of the M-63 group was higher than that of the placebo group.

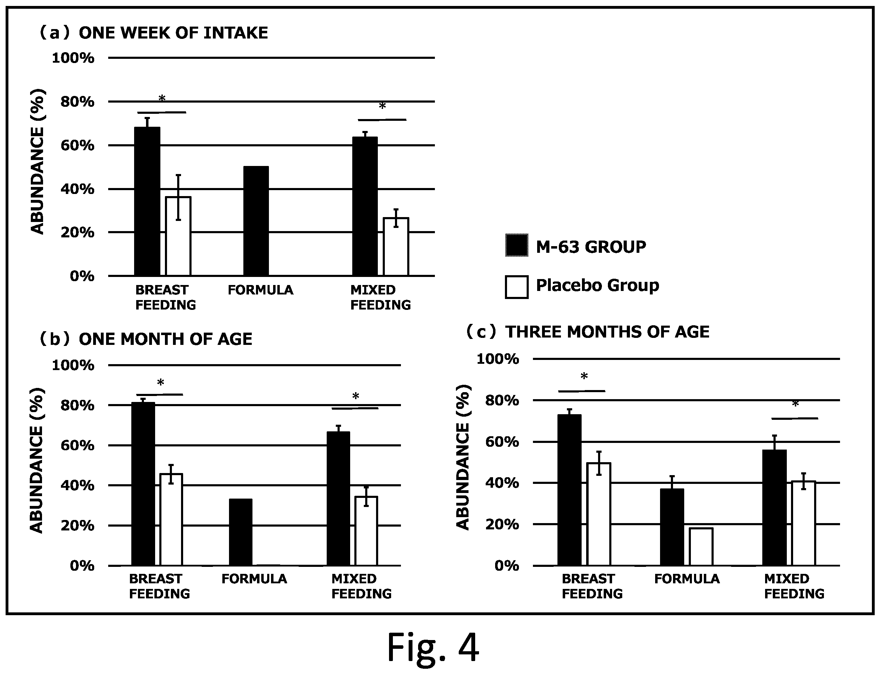

As shown in Fig. 4, the abundance of the Bifidobacterium of the M-63 group was higher than that of the placebo group, irrelevant to the nursing method of the infant, one week after starting the intake, one month of age and three months of age.

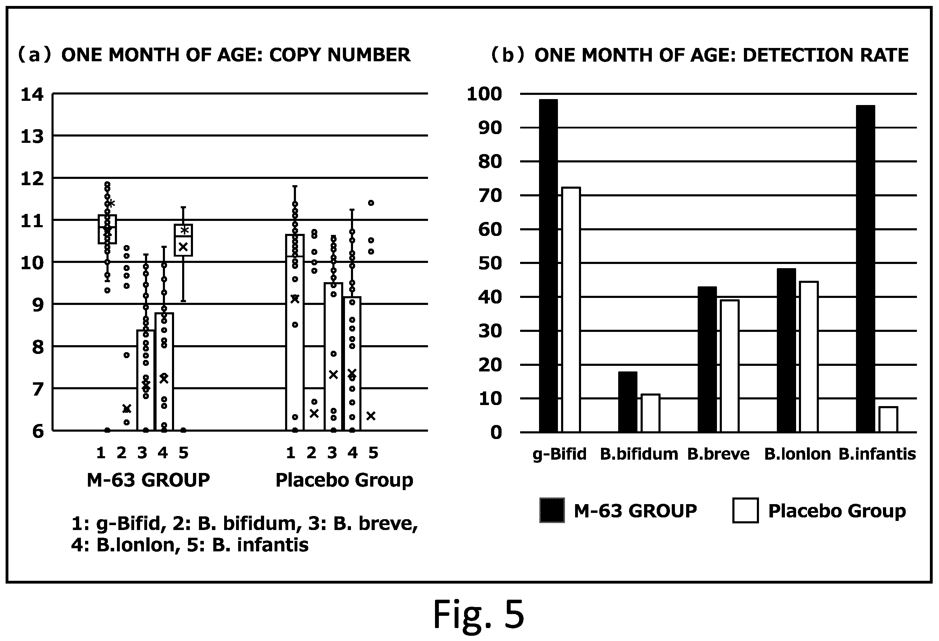

As shown in Fig. 5, the detection rate of *Bifidobacterium infantis* was significantly high (p<0.05) in the M-63 group one month of age.

### (2) Physicochemical Analysis of Feces

The short-chain fatty acid concentrations, the pH, the IgA amounts and the cytokine amounts of the fecal samples were measured by general methods.

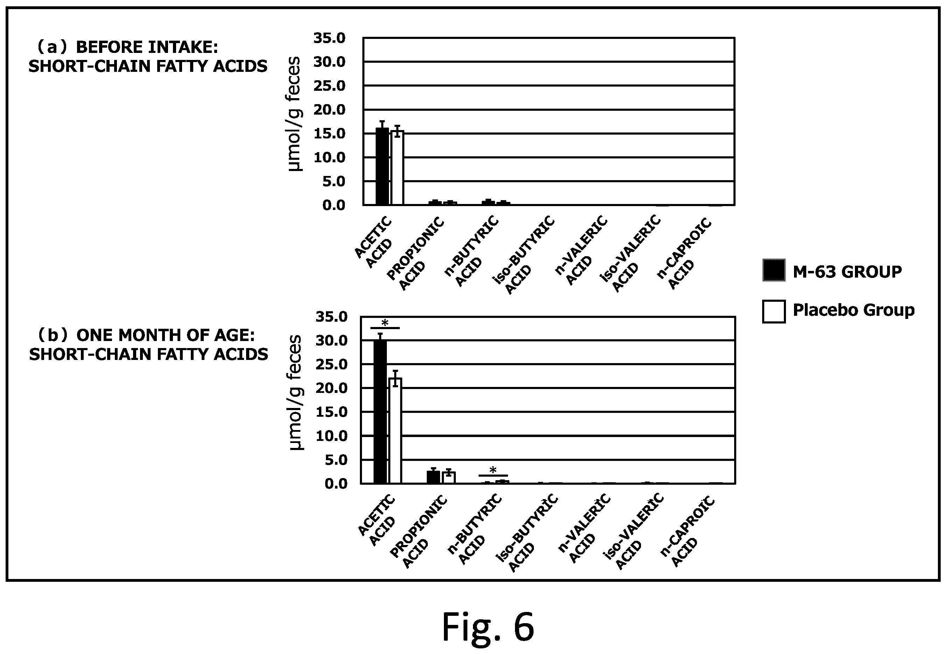

The results are shown in Figs. 6 to 8 and Table 1.

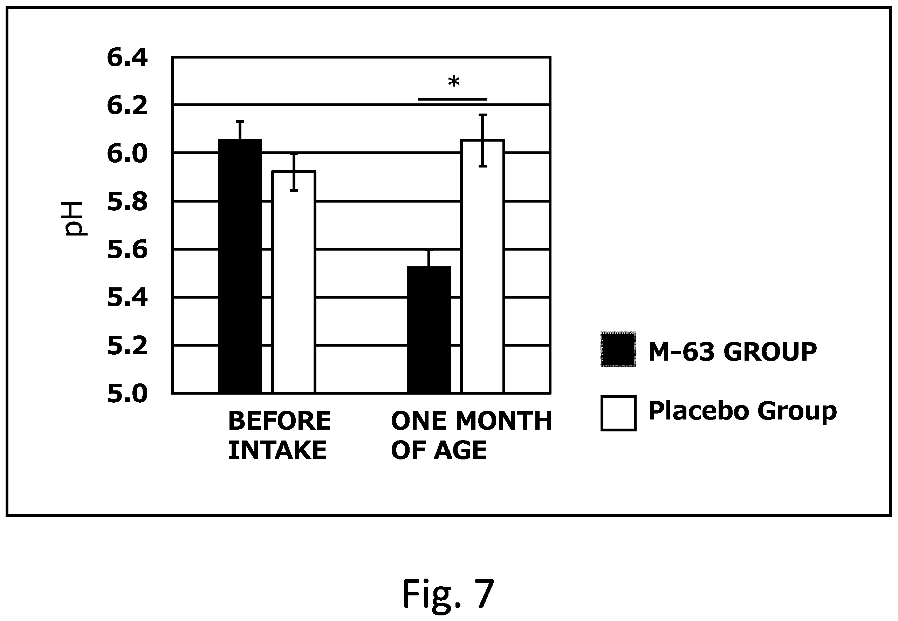

As shown in Figs. 6 and 7, one month of age, the acetic acid concentration of the M-63 group was significantly high, and the butyric acid concentration was significantly low. Moreover, the pH decreased largely. It was suggested that acetic acid produced by the increase in the Bifidobacterium decreased the intestinal pH.

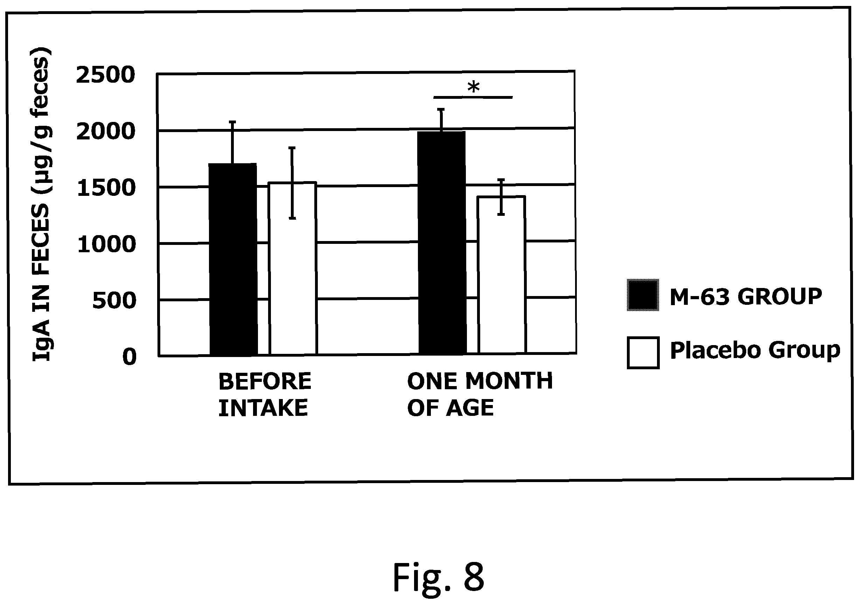

As shown in Fig. 8, the IgA amount in the feces increased significantly in the M-63 group one month of age. It was speculated that the intestinal microbiota was changed by the intake of *B. infantis* M-63 and that the secretion of IgA from the intestinal mucosa was thus promoted.

As shown in Table 1, the inflammatory cytokine amounts in the feces decreased in the M-63 group one month of age. It was suggested that an anti-inflammatory action can be exhibited in the intestines through the change in the intestinal microbiota caused by the intake of *B. infantis* M-63.

**[Table 1]**

| Table 1: Cytokine Amounts in Feces | | | | | | |
|---|---|---|---|---|---|---|
| Cytokine | M-63 Intake Group | | Placebo Group | | *P* value (Pre) | *P* value (1M) |
| | Median (IQR), pg/g protein in feces | | Median (IQR), pg/g protein in feces | | | |
| | Pre | 1M | Pre | 1M | | |
| IFN-γ | 141.0 (97.6-320.8) | 141.9 (80.9-266.4) | 222.4 (129.6-462.4) | 166.4 (109.8-399.1) | 0.104 | 0.255 |
| IL-1β | 1248.5 (389.4-5778.0) | 260.7 (108.5-759.1) | 4034.1 (1252.7-14812.3) | 629.1 (20.5.9-5495.8) | 0.049 | 0.003 |
| IL-2 | 27.6 (17.2-61.5) | 21.4 (13.5-35.4) | 58.7 (25.0-75.0) | 38.6 (13.6-61.6) | 0.167 | 0.047 |
| IL-5 | 19.1 (8.6-27.9) | 20.6 (7.8-44.9) | 19.9 (10.9-50.8) | 40.0 (18.5-72.6) | 0.532 | 0.032 |
| IL-6 | 34.3 (14.2-50.8) | 22.7 (8.5-35.9) | 25.4 (19.3-42.9) | 47.1 (21.3-63.7) | 0.783 | 0.009 |
| IL-8 | 141.9 (83.5-528.1) | 49.5 (13.5-111.5) | 137.6 (67.3-489.0) | 90.3 (45.5-388.6) | 0.699 | 0.010 |
| IL-10 | 2.8 (1.3-6.5) | 4.8 (1.4-7.9) | 3.4 (2.0-5.5) | 6.0 (2.2-11.0) | 0.655 | 0.109 |
| IL-22 | 4.1 (2.7-9.3) | 3.1 (1.3-6.8) | 5.6 (3.3-11.7) | 12.8 (3.4-21.7) | 0.224 | 0.005 |
| TNF-α | 25.0 (12.5-36.5) | 18.8 (10.9-38.8) | 26.6 (15.3-46.3) | 32.1 (13.5-59.5) | 0.209 | 0.090 |

| | | | | | | |
|---|---|---|---|---|---|---|
| Statistically significant values are in red P < 0.05 by *Mann-Whitney's* U test | | | | | | |

### (3) Frequency of Bowel Movements/Stool Consistency

The fecal states were evaluated from the collected fecal samples and the questionnaires on the subject children.

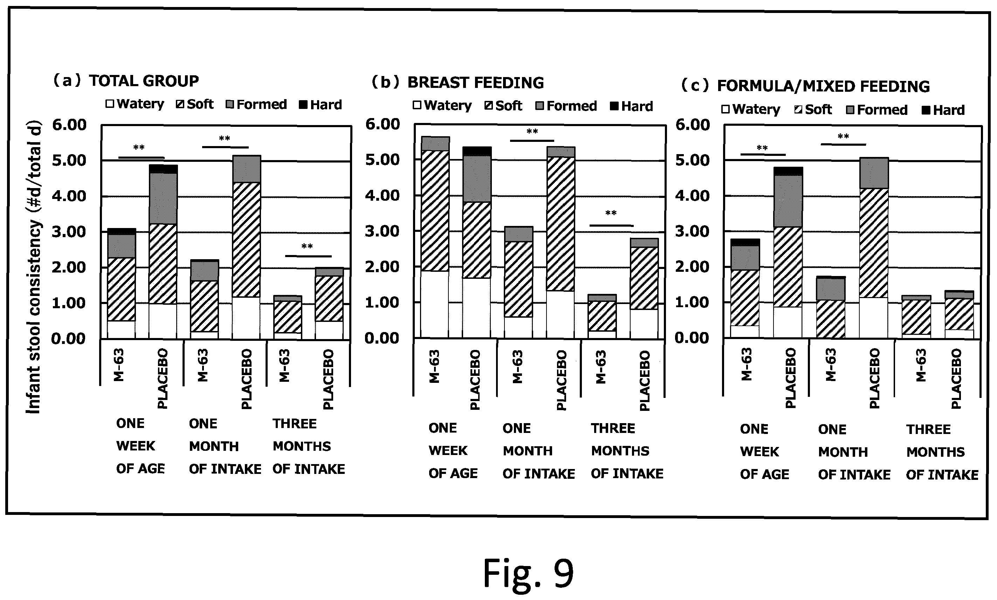

The results are shown in Fig. 9.

Both in the M-63 group and the placebo group, the frequency of bowel movements and watery stools decreased until three months of age.

The frequency of bowel movements was significantly lower in the M-63 group than that of the placebo group although the frequency was in the normal range.

It was suggested that maturation of intestinal functions such as water absorption and nutrient metabolism in the intestines was promoted by the intake of *B. infantis* M-63 and that the stool consistency and the frequency of bowel movements thus changed.

### (4) Frequencies of Vomiting and Milk Regurgitation

From the questionnaires on the subject children, the states of vomiting and milk regurgitation were evaluated.

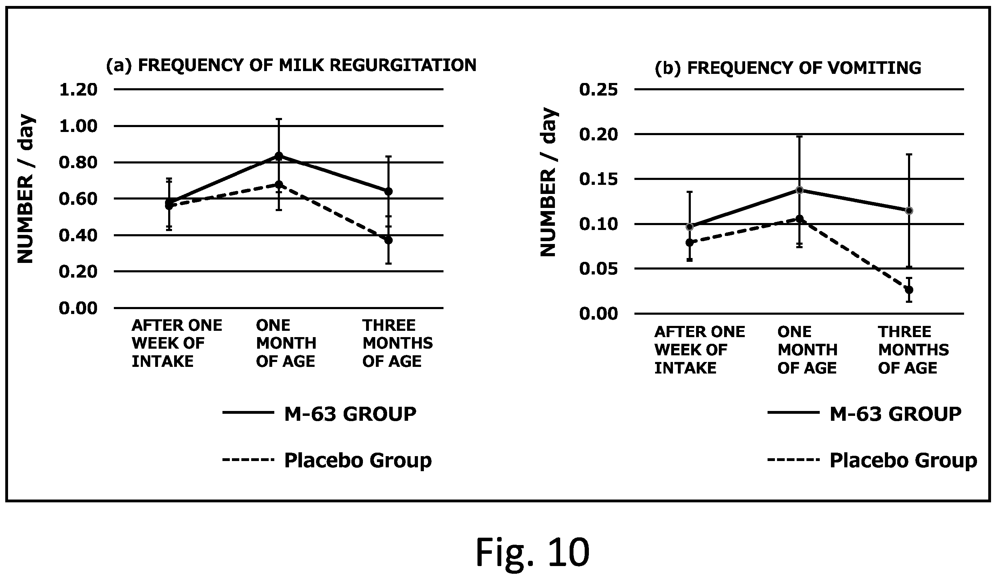

As in the results shown in Fig. 10 and Tables 2 and 3, no difference was found between the groups regarding vomiting, but the number of individuals with milk regurgitation of the M-63 group was significantly lower than that of the placebo group one week after starting the intake.

**[Table 2]**

| Table 2: Numbers of Individuals with Milk Regurgitation | | | |
|---|---|---|---|
| | After One Week of Intake | One Month of Age | Three Months of Age |
| M-63 Intake Group (n=56) | 32 | 31 | 29 |
| Placebo Group (n=53) | 41 | 34 | 23 |
| p value | p<0.05 | | |

**[Table 3]**

| Table 3: Numbers of Individuals with Vomiting | | | |
|---|---|---|---|
| | After One Week of Intake | One Month of Age | Three Months of Age |
| M-63 Intake Group (n=56) | 14 | 16 | 12 |
| Placebo Group (n=53) | 16 | 19 | 5 |

### (5) Period/Frequency of Continuous Crying

From the questionnaires on the subject children, the states of continuous crying were evaluated.

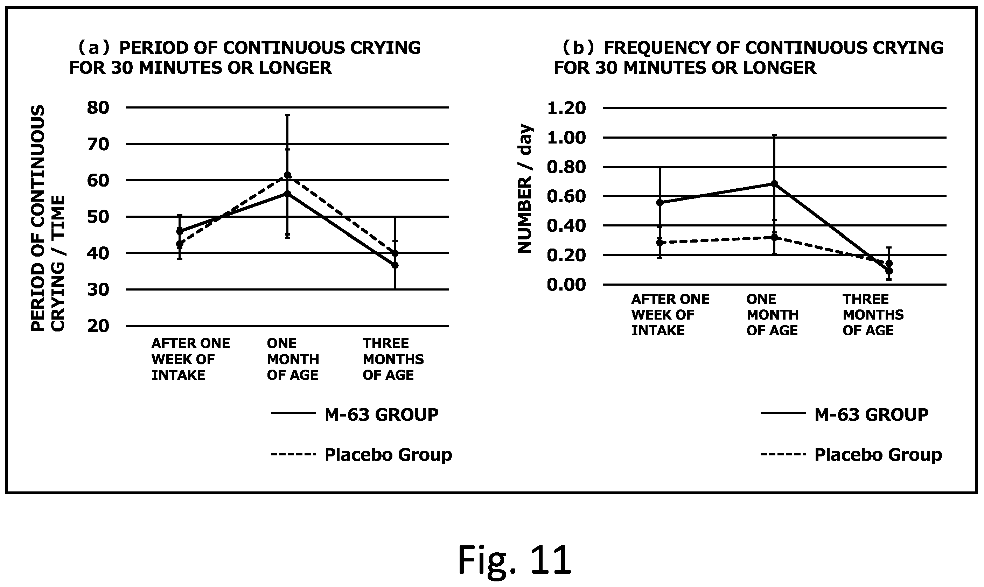

The results are shown in Fig. 11 and Table 4. No significant difference between the groups was found regarding the period and the frequency of continuous crying.

**[Table 4]**

| Table 4: Numbers of Individuals with Continuous Crying for 30 Minutes or Longer | | | |
|---|---|---|---|
| | After One Week of Intake | One Month of Age | Three Months of Age |
| M-63 Intake Group (n=56) | 9 | 9 | 3 |
| Placebo Group (n=53) | 10 | 11 | 3 |

### (6) Postpartum Depression Score

From the results of the answers based on the Postnatal Depression Scale (EPDS) by the mothers (women after delivery) of the subject children, the postpartum depression scores were calculated and evaluated.

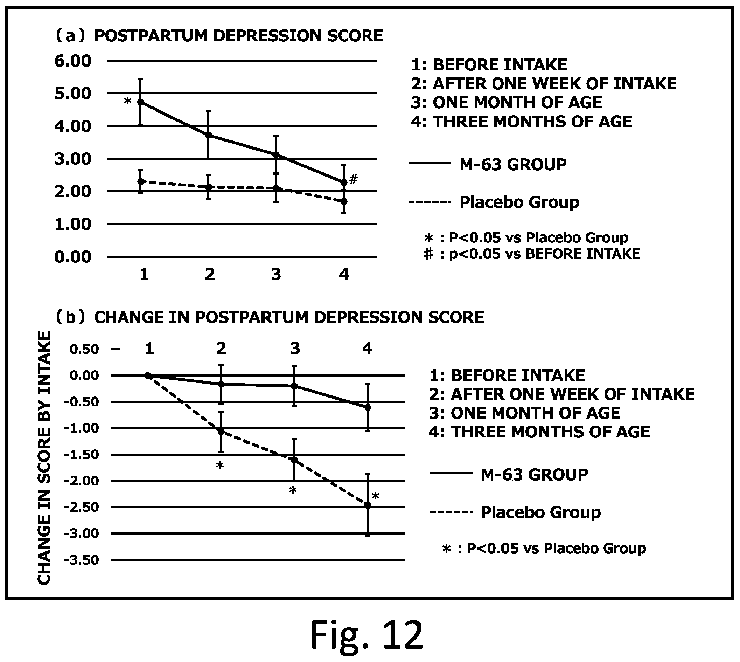

The results are shown in Fig. 12.

The postpartum depression scores of the **M-63** group were significantly higher than those of the placebo group before the intake, but there was no significant difference between the groups through the analysis of covariance using the values before the intake as the covariates. One week after starting the intake, one month of age and three months of age, the postpartum depression scores decreased significantly in the M-63 group compared to those of the placebo group.

### <Production Examples>

Production Examples of the compositions of the embodiments are shown below, but the compositions of the embodiments are not limited thereto.

### [Production Example 1]

*B. infantis* M-63 is added to 3 mL of an MRS liquid medium (DifcoTM Lactobacill MRS Broth, type number 288130) and anaerobically cultured at 32 to 39°C for five to 25 hours, and then the culture solution is concentrated and lyophilized to obtain lyophilized powder of the bacterium (namely bacterial powder). The bacterial powder and whey protein concentrate (WPC) are mixed evenly, and thus a composition is obtained. The composition in an amount of 20 g is dissolved in 200 g of water, and thus a composition containing M-63 is obtained. The obtained composition can be used as a composition for promoting colonization of a strain of *Bifidobacterium* in an intestinal microbiota, a composition for promoting maturation of an intestinal function, a composition for suppressing milk regurgitation or a composition for regulating immune function.

### [Production Example 2]

*B. infantis* M-63 is added to 3 mL of an MRS liquid medium and anaerobically cultured at 32 to 39°C for five to 25 hours, and then the culture solution is concentrated and lyophilized to obtain lyophilized powder of the bacterium (namely bacterial powder). The bacterial powder and dry powder of milk protein concentrate (MPC480, manufactured by Fonterra Co-operative Group Limited, protein content of 80 mass%, casein protein:whey protein = about 8:2) are mixed evenly, and thus a composition is obtained. The composition in an amount of 20 g is dissolved in 200 g of water, and thus a composition containing M-63 is obtained. The obtained composition can be used as a composition for promoting colonization of a strain of *Bifidobacterium* in an intestinal microbiota, a composition for promoting maturation of an intestinal function, a composition for suppressing milk regurgitation or a composition for regulating immune function.

### [Production Example 3]

A production method of a powdered formula containing *B. infantis* M-63 is shown below.

In 300 kg of warm water, 10 kg of desalted cow's milk whey protein powder (manufactured by MILEI GmbH), 6 kg of cow's milk casein powder (manufactured by Fonterra Co-operative Group Limited), 48 kg of lactose (manufactured by MILEI GmbH), 920 g of a mineral mixture (manufactured by Tomita Pharmaceutical Co., Ltd), 32 g of a vitamin mixture (manufactured by Tanabe Seiyaku Co., Ltd.), 500 g of lactulose (manufactured by Morinaga Milk Industry Co., Ltd.), 500 g of raffinose (manufactured by Nippon Beet Sugar Manufacturing Co., Ltd.) and 900 g of galactooligosaccharide syrup (manufactured by Yakult Pharmaceutical Industry Co., Ltd.) were dissolved and further heated and dissolved at 90°C for 10 minutes, and after adding 28 kg of modified fats (manufactured by Taiyo Yushi Corp.), the mixture was homogenized. Then, after sterilization and concentration steps, the mixture was spray dried, and about 95 kg of a powdered formula was thus prepared. To the powdered formula, 100 g of bacterial powder of M-63 (1.8×10¹¹ CFU/g, manufactured by Morinaga Milk Industry Co., Ltd.) triturated in starch is added, and about 95 kg of a powdered formula containing the strain of *Bifidobacterium* and oligosaccharides is thus prepared. When the obtained powdered formula is dissolved in water and when a formula solution having a total solid content concentration as a standard formula concentration of 14% (w/V) is obtained, 10⁶ to 10⁹ CFU/100 ml can be obtained as the bifidobacteria count in the formula solution.

The powdered formula containing M-63 obtained in the above manner can be used as a composition for promoting colonization of a strain of *Bifidobacterium* in an intestinal microbiota, a composition for promoting maturation of an intestinal function, a composition for suppressing milk regurgitation or a composition for regulating immune function.

## Claims

1. A composition for promoting colonization of a strain of *Bifidobacterium* in an intestinal microbiota
which is a composition that comprises one kind or two or more kinds selected from bacterial cells of *Bifidobacterium longum* subsp. *infantis* M-63 (NITE BP-02623), a culture of the bacterium and a treated product of the bacterium and that is taken by a healthy full-term newborn, infant or small child.

2. A composition for promoting maturation of an intestinal function
which is a composition that comprises one kind or two or more kinds selected from bacterial cells of *Bifidobacterium longum* subsp. *infantis* M-63 (NITE BP-02623), a culture of the bacterium and a treated product of the bacterium and that is taken by a healthy full-term newborn, infant or small child.

3. A composition for suppressing milk regurgitation
which is a composition that comprises one kind or two or more kinds selected from bacterial cells of *Bifidobacterium longum* subsp. *infantis* M-63 (NITE BP-02623), a culture of the bacterium and a treated product of the bacterium and that is taken by a healthy full-term newborn, infant or small child.

4. A composition for regulating immune function
which is a composition that comprises one kind or two or more kinds selected from bacterial cells of *Bifidobacterium longum* subsp. *infantis* M-63 (NITE BP-02623), a culture of the bacterium and a treated product of the bacterium and that is taken by a healthy full-term newborn, infant or small child.

5. The composition according to any one of claims 1 to 4 which is taken at 1.0×10⁸ to 5.0×10⁹ cfu/kg body weight/day.

6. The composition according to any one of claims 1 to 4 which is taken in combination with breast feeding.

7. The composition according to any one of claims 1 to 4 which is a food or a drink.

8. The composition according to any one of claims 1 to 4 which is a pharmaceutical product.

9. A composition for promoting colonization of a strain of *Bifidobacterium* in the intestinal microbiota of a healthy full-term newborn, infant or small child, comprising one kind or two or more kinds selected from bacterial cells of *Bifidobacterium longum* subsp. *infantis* M-63 (NITE BP-02623), a culture of the bacterium and a treated product of the bacterium.
